# EUROPEAN PATENT SPECIFICATION

(11) **EP 2 171 025 B1**
(45) Date of publication and mention of the grant of the patent: **25.04.2012**
(21) Application number: 08770623.0
(22) Date of filing: 11.06.2008
(51) Int. Cl.: C11D 3/00, C11D 3/37, C11D 3/22, C11D 1/90, C11D 1/92, A61L 12/14

(54) **OPHTHALMIC COMPOSITION WITH HYALURONIC ACID**
OPHTHALMISCHE ZUSAMMENSETZUNG MIT HYALURONSÄURE
COMPOSITION OPHTALMIQUE COMPRENANT DE L'ACIDE HYALURONIQUE

(30) Priority: 13.06.2007 US 943620 P; 02.06.2008 US 131549
(43) Date of publication of application: 07.04.2010
(73) Proprietor: Bausch & Lomb Incorporated, Rochester, NY 14604-2701 (US)
(72) Inventor: BURKE, Susan, E., Batavia, NY 14020 (US); VENKATESH, Srini, Pittsford, NY 14534 (US); HEILER, David, Joseph, Avon, NY 14414 (US)
(74) Representative: Glas, Holger
(86) International application number: PCT/US2008/066464
(87) International publication number: WO 2008/157140

(56) References cited:
- WO-A-99/24543
- US-A1- 2005 074 467

## Description

The present invention relates to an ophthalmic composition with hyaluronic acid and a biguanide, and to a method of cleaning, disinfecting or packaging contact lenses with the composition.

### Background of the Invention

Biguanides, including polymeric biguanides, as a class are known to have antimicrobial activity. Poly(hexamethylene biguanide) also known as PHMB or PAPB has been used as an antimicrobial component in many applications including topical disinfectants and as a preservative in health care products. PHMB is commonly represented by the following formula, though it is known to exist as a complex mixture of polymeric biguanides with various terminal groups including guanidine (not shown).

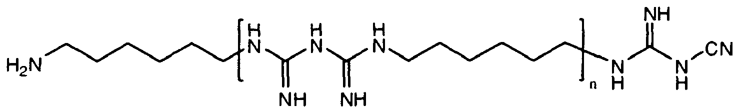

The value n represents the number of repeating units of the biguanide polymer. GB 1434040 describes the use of PHMB and several other biguanide structures and their effectiveness as antimicrobial components.

PHMB has been used in ophthalmic compositions, e.g., in contact, lens care solutions. Ophthalmic lens care solutions that contain PHMB represent a significant improvement in patient comfort and antimicrobial effectiveness compared to most other antimicrobial components. However, as with any antimicrobial component there remains a tradeoff between the concentration of the antimicrobial component in the solution and the comfort experienced by the patient. Due to its wide commercial acceptance, extensive efforts have been made to improve the antimicrobial efficacy or the comfort level to the patient by chemically modifiying PHMB. For example, many derivatives of PHMB have been reported that alter the length of the alkylene group, place substituents on the alkylene repeating unit, change the length of the polymer, i.e., molecular weight or n, or modify the terminal groups.

EP701821 describes a biguanide derivative having I to 500 polymeric repeat units that is formulated with a phosphoric acid and phosphoric acid salt. EP788797 describes the use of PHMB having a molecular weight of less than 5000 Da, e.g., 3000 Da to 4000 Da for treatment of urogenital disease and parasites of the abdominal cavity.

U.S. 6,121,327 describes a PHMB derivative in which the amino terminal groups are replaced by amido groups, RC(O)NH-. The R group can include alkyls, cycloalkyls, polyethyleneoxides and polypropyleneoxides. In the case of poly(ethyl)propylene oxides, the added surfactant character is said to provide good efficacy and lower toxicity.

U.S. 5,965,088 describes a PHMB derivative in which the terminal groups are replaced by a branched or unbranched alkyl, cycloalkyl, ether or sulfide having four to twelve carbon atoms. US 2005/0074467A1 discloses a liquid formulation for contact lenses which comprises polylysine, polyphosphonic acid and/or salt thereof as a substance for substance for suppressing the adhesion of polylysine to a contact lens, nitrogen-containing organic anti-microbial agent excluding polylysine and water.

Hyaluronic acid is a linear polysaccharide (long-chain biological polymer) formed by repeating disaccharide units consisting of D-glucuronic acid and N-acetyl-D-glucosamine linked by β(1-3) and β(1-4) glycosidic linkages. Hyaluronic acid is distinguished from the other glycosaminoglycans, as it is free from covalent links to protein and sulphonic groups. Hyaluronic acid is ubiquitous in animals, with the highest concentration found in soft connective tissue. It plays an important role for both mechanical and transport purposes in the body; e.g., it gives elasticity to the joints and rigidity to the vertebrate disks, and it is also an important component of the vitreous body of the eye.

Hyaluronic acid, because of its high degree of hydration, is likely responsible for increasing the resistance of biological tissues or cells to compression. This is a role based on hyaluronic acid's capacity to hold more water than any other natural or synthetic polymer. Also, the viscoelastic properties of hyaluronic acid, that is, hard elastic under static conditions though less viscous under small shear forces enables hyaluronic acid to basically function as a shock absorber for cells and tissues. The hyaluronic acid properties are dependent on the molecular weight, the solution concentration, and physiological pH. In low concentrations, the individual chains entangle and form a continuous network in solution, which gives the system interesting properties, such as pronounced viscoelasticity and pseudoplasticity that is unique for a water-soluble polymer at low concentration.

There remains an interest and need for an improved antimicrobial biguanide composition that offers a greater comfort level to the patient without sacrificing antimicrobial efficacy.

### Summary of the Invention

The invention is directed to an ophthalmic composition comprising a polymeric biguanide composition having less than 18 mol% of terminal amine groups and 55 mol% or greater of terminal guanidine groups as measured by ¹³C NMR, and 0.005 w/v% to 0.04 w/v% hyaluronic acid.

The invention is also directed to an ophthalmic composition comprising a polymeric biguanide composition comprising less than 18 mol% of terminal amine groups and 40 mol% or greater of terminal cyanoguanidino groups as measured by ¹³C NMR, and 0.005 w/v% to 0.04 w/v% hyaluronic acid.

The invention is also directed to a method of cleaning, disinfecting or packaging a contact lens with the ophthalmic compositions

### Brief Description of the Figures

Figure 1 is a ¹³C NMR spectrum of PHMB-CG;

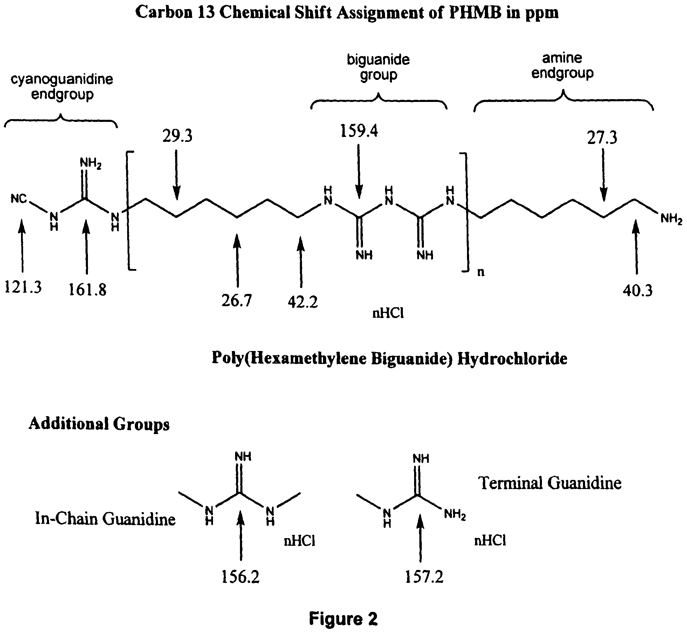

Figure 2 shows the assigned peak assignments of a ¹³C NMR spectrum of a commercial sample of PHMB; and
Figure 3 shows the median comfort profile of an ophthalmic solution of the invention verses Opti-Free Replenish®, a commercial solution from Alcon Laboratories, Inc.

### Detailed Description of the Invention

PHMB is a mixture of various biguanide polymers that can include different combinations of terminal groups, e.g., amine, cyanoguanidino, and guanidine. Based only on these three terminal groups, at least six possible biguanide polymers can exist. There can be one biguanide polymer with two terminal amine groups, which we refer to as PHMB-AA, one with two terminal cyanoguanidino groups, which we refer to as PHMB-CGCG, and one with two terminal guanidine groups, which we refer to as and PHMB-GG (see, below). There are also the three possible biguanide polymers having a combination of two different terminal groups. Again, based on the above terminal groups they include amine-cyanoguanidino (PHMB-ACG), amine-guanidino (PHMB-AG) and guanidine-cyanoguanidino (GCG). Accordingly, a commercial sample of PHMB will likely comprise a mixture of polymeric biguanides with the three mentioned terminal groups though how these terminal groups are arranged on each polymer and what the molar concentration of each type of terminal groups is in the mixture provides a relatively complex picture of the polymeric biguanide composition. Moreover, some of the composition can include in-chain polymeric guanide (not shown). The subscript "n" represents the average number of repeating groups, and a distribution of polymer length exists for each of the polymers shown below

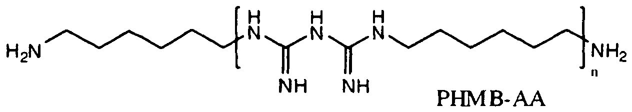

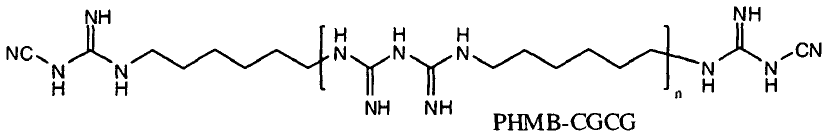

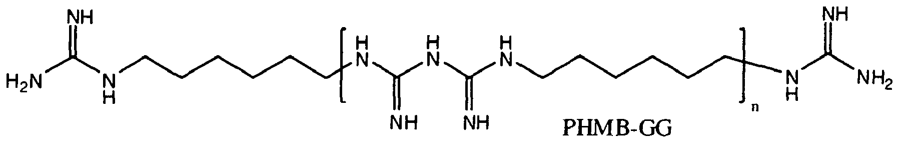

Using ¹³C NMR we have estimated the molar concentration of terminal amine groups in commercial PHMB (Cosmocil®-type PHMB) to range from 20% to 30%. Similarly, we have estimated the molar concentration of terminal guanidine groups and terminal cyanoguanidino groups to range from 38% to 49% and 30% to 32%, respectively (see, Table 1).

In contrast, the polymeric biguanide present in the compositions of the invention are characterized by a relatively low molar concentration of terminal amine groups than the commercial samples of PHMB, that is, Cosmocil® type PHMB. The polymeric biguanide compositions are also characterized by a relatively high molar concentration of terminal guanidine groups or terminal cyanoguanidino groups than the commercial samples of PHMB. At times, we shall refer to these novel polymeric biguanide compositions in this patent application as PHMB-CG.

The ophthalmic compositions of the invention also includes hyaluronic acid or the corresponding metal salts including, for example, sodium hyaluronate (the sodium salt), potassium hyaluronate, zinc hyaluronate, magnesium hyaluronate, and calcium hyaluronate (hereafter, collectively as hyaluronic acid). It is well understood by one of ordinary skill in the art that the term "hyaluronic acid" includes the corresponding metal salts of the acid form.

Hyaluronic acid is a natural polymer comprising repeating disaccharide units (glucuronic acid and N-acetyl glycosamine). Hyaluronic acid is produced in the body by connective tissue cells of most animals, and is present in large amounts in such tissues as the vitreous humor of the eye and the synovial fluids of joints.

Hyaluronic acid can be isolated from a variety of natural sources and is commercially available from various commercial suppliers. In its natural form, hyaluronic acid has a molecular weight in the range of 5 x 10⁴ up to 1 x 10⁷ daltons. Its molecular weight may be reduced via a number of cutting processes such as exposure to acid, heat (e.g. autoclave, microwave, dry heat) or ultrasound.

Alternatively, hyaluronic acid can be prepared by fermentation of bacteria such as streptococci. The bacteria are incubated in a sugar rich broth, and the produced hyaluronic acid is separated from impurities and purified. The molecular weight of hyaluronic acid produced via fermentation can be set by the sugars placed in the fermentation broth. Hyaluronic acid produced via fermentation is commercially available.

### PHMB-CG

In one instance, PHMB-CG comprises less than 18 mol% of terminal amine groups, and 55 mol% or greater of terminal guanidine groups as measured by ¹³C NMR. In another instance, PHMB-CG comprises less than 15 mol%, or less than 10 mol%, of terminal amine groups, and 60 mol% or greater, or 65 mol% or greater, of terminal guanidine groups, as measured by ¹³C NMR. The term "measured by ¹³C NMR" means to relatively quantify specific carbon peaks associated with each type of terminal group or in-chain biguanide/guanide for a polymeric biguanide composition using the pulse technique described in this application under the subheading Examples.

Alternatively, PHMB-CG can be described as a polymeric biguanide composition that comprises 55 mol% to 90 mol% terminal guanidino groups, 5 mol% to 35 mol% terminal cyanoguanidino groups and less than 18 mol% terminal amine groups, as measured by ¹³C NMR. In another instance, PHMB-CG comprises 60 mol% to 90 mol% terminal guanidino groups, 8 mol% to 25 mol% terminal cyanoguanidino groups and less than 12 mol% terminal amine groups, as measured by ¹³C NMR.

Alternatively, PHMB-CG can be described as a biguanide composition that comprises polymeric biguanides of formula (1), formula (2). formula (3) and optionally formula (4), and has a molar ratio of
[mol% formula (1) + mol% formula (2)]:[mol% formula (3) + mol% formula (4)] from 70:30 or greater, as measured by ¹³C NMR 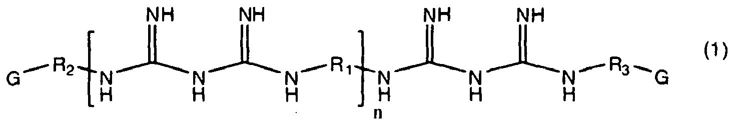 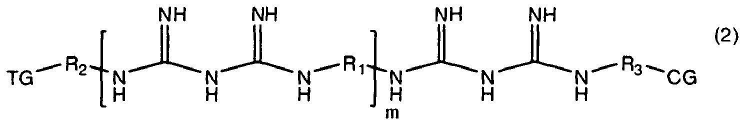 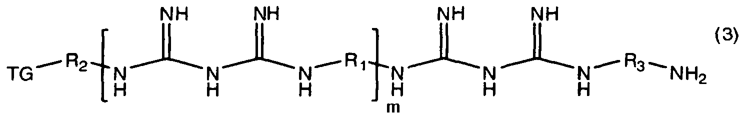 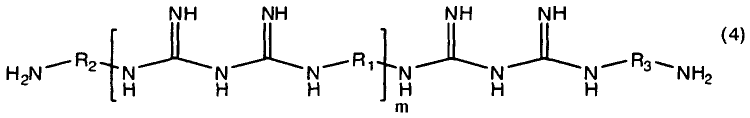 wherein CGis 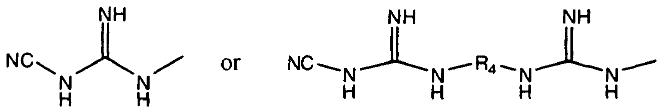 G is 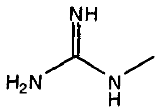 and each TG is the same or different and is selected from CG or G. R₁, R₂ and R₃ are divalent radicals of an aliphatic hydrocarbon independently selected from the group consisting of a C₃-C₁₂ alkylene, C₄-C₁₂ oxyalkylene and C₄-C₁₂ thioalkylene. In one embodiment, R₁, R₂ and R₃ are independently selected from a C₄-C₈ alkylene. R₄ is selected from the group consisting of a C₂-C₁₂ alkylene, C₄-C₁₂ oxyalkylene and C₄-C₁₂ thioalkylene, preferably a C₄-C₁₂ alkylene. The value n represents a number average of repeat units between I and 20, and the value m is independently selected for each of formulas (2), (3) and (4) and represents a number average of repeat units between I and 20.

It is believed that the increase in terminal guanidine groups results in part from the cleavage of the in-chain biguanide over time during preparation of the polymeric biguanide composition. This is supported in-part by the observed decrease of the in-chain biguanide content as the preparation heating time is increased from one to four hours at the same temperature.

Ordinarily, with cleavage of the in-chain biguanide one should observe a decrease in the number average molecular weight (M_{N}) of the biguanide composition. We observe, however, a slight increase in M_{N}, which is also dependent upon the preparation heating time (longer heating times results in higher M_{N}). These observations suggest that the preparation may also involve formation of in-chain biguanide by the reaction of a terminal amine group of one polymer with a terminal cyanoguanidino group of another polymer, and thus, resulting in a composition of slightly higher M_{N}. In combination, these two processes will tend to lower the mol% of terminal amine groups and increase the mol% of terminal guanidine groups.

In addition, as a result of the two above described processes, one would expect that on average, polymers of formula (1) will likely have a lower M_{N}, and consequently, the average value of n in formula (1) should be lower than the average value of m in formula (2) or formula (3).

Alternatively, PHMB-CG can be described as a biguanide composition that comprises polymeric biguanides of formula (1) and formula (2) 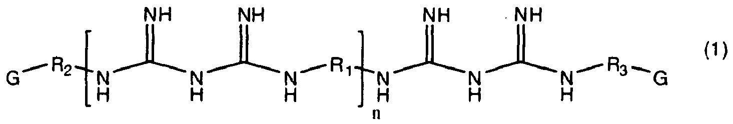 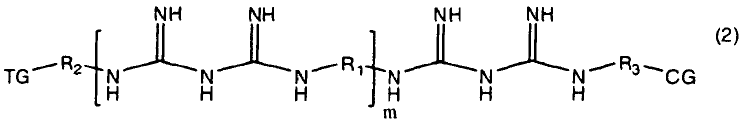 wherein the polymeric biguanides of formula (I) and formula (2) account for at least 80 mol%, or at least 90 mol%, of the total moles of polymeric biguanides in the composition, as measured by ¹³C NMR, wherein CGis 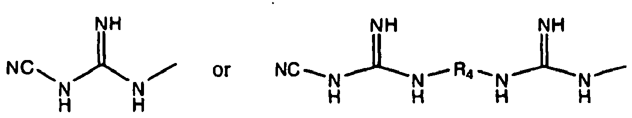 G is 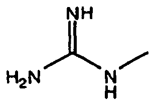 and each TG is the same or different and is selected from CG or G;
R₁, R₂ and R₃ are divalent radicals of an aliphatic hydrocarbon independently selected from the group consisting of a C₃-C₁₂ alkylene, C₄-C₁₂ oxyalkylene and C₄-C₁₂ thioalkylene;
R₄ is selected from the group consisting of a C₂-C₁₂ alkylene, C₄-C₁₂ oxyalkylene and C₄-C₁₂ thioalkylene, preferably a C₄-C₁₂ alkylene; and
n and m represent a number average of repeat units between I and 20. Again, for the reasons described above, one can expect the average value of n in formula (1) should be lower than the average value of m in formula (2).

Alternatively, PHMB-CG can be described as a biguanide composition that comprises less than 18 mol% of terminal amine groups and 40 mol% or greater of terminal cyanoguanidino groups as measured by ¹³C NMR. The polymeric biguanide composition also is characterized by a relative decrease in the molar concentration of terminal guanidine groups. This biguanide composition is prepared by using a similar synthetic route as that of conventional PHMB, e.g., the preparation of Cosmocil® type PHMB, with the exception that one adds from 15% to 40% by weight of a cyanoguanidino agent, e.g., hexamethylene bis(cyanoguanidino) (HMBDA), to the reaction mixture.

In one instance, PHMB-CG comprises less than 15 mol% of terminal amine groups, and 45 mol% or greater, or 50 mol% or greater, of terminal cyanoguanidino groups, as measured by ¹³C NMR. Also, the biguanide composition will comprise from 10 mol% to 30 mol% of terminal guanidine groups, as measured by ¹³C NMR. In another instance, PHMB-CG comprises 45 mol% to 70 mol% terminal cyanoguanidino groups, 10 mol% to 30 mol% of terminal guanidine groups and 7 mol% to 15 mol% terminal amine groups, as measured by ¹³C NMR.

The polymeric biguanide composition with the relatively high terminal cyanoguanidino groups is typically characterized by in-chain biguanide concentration of 90 mol% or greater, or 92 mol% or greater, as measured by ¹³C NMR, which is similar to, and typically greater than that observed in commercial PHMB (89 mol% to 92 mol%). One particular preparation provided an in-chain biguanide concentration of about 95 mol%.

Similar to the first described biguanide composition, the polymeric biguanide composition with high terminal cyanoguanidino groups can comprise polymeric biguanides of formula (1) and formula (2) 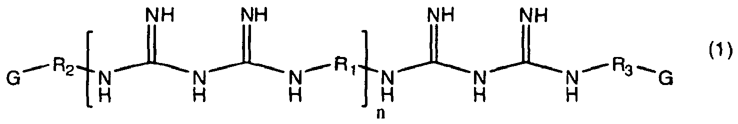 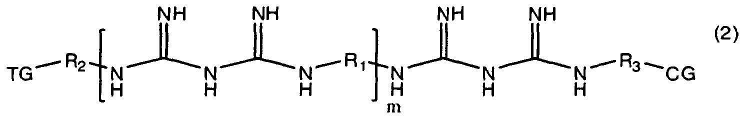 wherein the polymeric biguanides of formula (1) and formula (2) account for at least 80 mol% of the total moles of polymeric biguanides in the composition, as measured by ¹³C NMR, wherein CG is 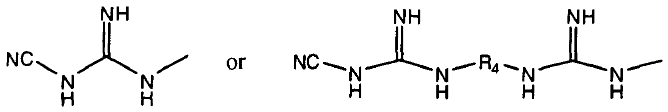 G is 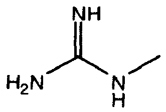 and each TG is the same or different and is selected from CG or G;
R₁, R₂ and R₃ are divalent radicals of an aliphatic hydrocarbon independently selected from the group consisting of a C₃-C₁₂ alkylene, C₄-C₁₂ oxyalkylene and C₄-C₁₂ thioalkylene;
R₄ is selected from the group consisting of a C₂-C₁₂ alkylene, C₄-C₁₂ oxyalkylene and C₄-C₁₂ thioalkylene, preferably a C₄-C₁₂ alkylene; and
n and m represent a number average of repeat units between I and 20.

The M_{N} of the biguanide polymers compositions of the inventions will range from 700 Da to 12,000 Da, from 1000 Da to 8,000 Da, or from 1000 Da to 4000 Da. Accordingly, the average values of m, n and p, and R₁, R₂ and R₃ are selected to provide biguanide polymers within this range of average number molecular weight.

Any one of the above PHMB-CG compositions can be used as an antimicrobial component in an ophthalmic composition of the invention with hyaluronic acid. For example, the ophthalmic compositions can be used as a component in a contact lens solution to clean, disinfect or package the lens. Alternatively, the ophthalmic composition can be as a preservative in a pharmaceutical formulation that includes a pharmaceutical agent.

As used herein, the term "ophthalmic composition" defines a composition intended for application in the eye or intended for treating a device to be placed in contact with the eye such as a contact lens. Ophthalmic compositions can include compositions for direct placement in the eye, including eye drop solutions such as for treating dry eye, and contact lens treating solutions. Ophthalmic compositions also include those compositions formulated as multi-purpose solutions for cleaning and disinfecting contact lenses or to package contact lens.

The term "preservative" or "to preserve" refers to the use of the compositions for the purpose of inhibiting the growth of microorganisms in a particular product, e.g., in an eye drop formulation.

### Preparation of the PHMB-CG Compositions

The PHMB-CG biguanide can be prepared from commercially available polymeric biguanide compositions. For example, PHMB can be used as a starting material to which is added a cyanoguanidino agent, guanidine agent or a mixture thereof in the presence of a mineral acid or an organic acid. The resulting product is characterized by an increase concentration of guanidine or cyanoguanidino terminal groups at the expense of the amino terminal groups. The amount of cyanoguanidino agent or guanidine agent added will depend upon the desired degree of cyanoguanidino(guanidine)/amine exchange. Theoretically, to exchange most, if not all, of the amine terminal groups in a commercial sample of PHMB, one would add approximately four molar equivalents of cyanoguanidino agent, guanidine agent or a mixture thereof for each mole equivalent of PHMB as there are about four molar equivalents of terminal amine groups for every mole of PHMB.

In one embodiment, a reaction mixture is prepared by grinding together fine particles of a commercial sample of PHMB and a bis-cyanoguanidino alkane. The ground mixture is added to a reaction flask along with a small amount of acid, e.g., a mineral acid or an organic acid, to facilitate the exchange reaction. The reaction flask is heated to 120°C or more, e.g., above 140 °C or 150°C, for a period of about one to about four hours. It is to be understood by one of ordinary skill, that in general, lower reaction temperatures would require longer reaction times. The reaction mixture is cooled and the resulting solids dissolved in a first solvent and then precipitated by the addition of a second solvent. For example, the first solvent can be water and the second solvent can be acetone.

Alternatively, one can use a dicyanamide such as sodium dicyanamide or zinc dicyanamide as the cyanoguanidino agent. Again, to theoretically exchange all of the amino terminal groups one would add about four molar equivalents of the dicyanamide for each molar equivalent of commercial PHMB. The PHMB and dicyanamide are added to a reaction vessel and heated. After heating at about 150°C for about one to about four hours the reaction is cooled to room temperature under nitrogen overnight. The resulting solids are dissolved in water and reprecipitated with acetone.

Dialysis can be used as an alternative approach to remove reaction impurities and undesired, low molecular weight products and reactants from the resulting reaction solids. In this case, the reaction solids are dissolved in water and the solution undergoes dialysis (100 MWCO tubing) overnight. The resulting product is then freeze-dried.

In another embodiment, the polymeric biguanide compositions can be prepared using a modified synthetic preparation of commercial PHMB. In this case, approximately, 10 mol% to 50 mol%, or 20 mol% to 40 mol% (based on the moles of diamine), of the cyanoguanidino agent, guanidine agent or mixture thereof, is added to the reaction mixture. A preparation of PHMB is reported in U.S. Patent No. 3,428,576 (Examples 1 to 3).

### Hyaluronic Acid

The isolation of hyaluronic acid from rooster combs typically includes an enzymatic digestion followed by one or more separation steps to remove proteins and provide a crude extract. Additional purification steps include precipitation in ethanol and redissolution in sodium chloride solution. Thus, a typical process for isolating hyaluronic acid from rooster comb includes removal of epithelium from the combs, grinding of combs, treatments in acetone and multiple treatments with ethanol and sodium chloride solutions. Several U.S. patents describe methods to isolate and purify hyaluronic acid including U.S. Pat. Nos. 4,141,973; 4,784,990; 5,099,013; 5,166,331; 5,316,926; 5,411,874; 5,559,104 and 5,925,626.

The hyaluronic acid used to prepare the compositions was obtained via a fermentation process and commercially supplied from Shandong Freda Biochem Co. Ltd., China. The hyaluronic acid produced by fermentation can have several commercial advantages over hyaluronic acid produced from extraction and purification of natural sources. Hyaluronic acid obtained from a fermentation mixture comprising Streptococcus equi is particularly advantageous. Also, it is advantageous that the hyaluronic acid have a glucuronic acid content that is greater than 42% by weight.

The hyaluronic acid is present in the ophthalmic compositions of the invention over a relatively limited concentration range from 0.005 wt.% to 0.04 wt.%. In many of the compositions, the hyaluronic acid concentration is from 0.01 wt.% to 0.025 wt.%. If the concentration of the hyaluronic acid is below 0.002 wt.% the commercial advantages of improved patient comfort is virtually non-existent. If on the other hand, the hyaluronic acid concentration is too high relative to the amount of PHMB present, e.g., if the hyaluronic acid concentration is about 0.02 wt.% and the PHMB concentration is about 1 to about 1.3 ppm (a calculated weight ratio of hyaluronic acid to PHMB of about 150 to 200), one begins to notice a decrease in the biocidal efficacy of the compositions over time, and in particular, with respect to the microorganism, *C. albicans.* In many of the compositions, the hyaluronic acid concentration is from 0.0075 wt.% to 0.015 wt% and the PHMB concentration is from 0.8 ppm to 2.0 ppm, though one must keep in mind a weight ratio of the two solution components as explained below.

The weight ratio of hyaluronic acid to PHMB is critical to maintaining patient comfort and biocidal efficacy over an extended period of time at 30 °C. If the weight ratio of hyaluronic acid to PHMB is above 120, one begins to observe a decrease in biocidal efficacy over time though patient comfort is acceptable. On the other hand, if the weight ratio hyaluronic acid to PHMB is below 45, one begins to notice a decrease in patient comfort though the compositions are able to maintain the requisite biocidal properties for many months at 30 °C. One of the preferred weight ratios of hyaluronic acid to PHMB is from 55:1 to 90:1. Still another preferred weight ratio of hyaluronic acid to PHMB is from 60:1 to 80:1.

### 1. PHMB-CG/HA Compositions and the Use Thereof In Antimicrobial Formulations

The compositions of the invention can be used for eye drops, ophthalmic solutions, gels or ointments. In particular, the compositions can be formulated as an antimicrobial component for an ophthalmic lens care solution, which can be used to clean, disinfect or package contact lenses. In this case, the PHMB-CG/HA compositions will be formulated with a number of other solution components that provide additional properties required of such solutions.

The PHMB-CG/HA compositions can be formulated with other cationic antimicrobial components. Suitable antimicrobial components include, but are not limited to, quaternary salts used in ophthalmic applications such as cetylpyridinium chloride, α-[4-tris(2-hydroxyethyl)ammonium chloride-2-butenyl]poly[1-dimethylammonium chloride-2-butenyl]-ω-tris(2-hydroxyethyl)ammonium chloride (available as Polyquaternium-1®). Polyquaternium-42, often referred to as polixetonium (see, U.S. Patent No. 5,300,296 is another polyquaternium of particular interest. Polixetonium is present in the compositions from 1 ppm to 10 ppm. For example, a preferred composition will comprise from 0.6 ppm to 1.2 ppm PHMB-CG and from 1.5 ppm to 4 ppm polixetonium. Other cationic antimicrobial components include enzalkonium halides, and biguanides such as salts of alexidine, alexidine-free base, salts of chlorhexidine, antimicrobial polypeptides and mixtures thereof.

Exemplary cationic disinfecting antimicrobial component used in combination with PHMB-CG is cetylpyridinium chloride or polyquaternium-1. For example, a preferred composition will comprise from 0.4 ppm to 1.1 ppm PHMB-CG and from 0.2 ppm to 0.8 ppm cetylpyridinium chloride or from 0.4 ppm to 1.1 ppm PHMB-CG and from I ppm to 3 ppm polyquaternium-1. The term "cationic" when referring to an antimicrobial component refers to the predominant form of the antimicrobial component at neutral pH having a positive charge and a counteranion.

The lens care solutions will very likely comprise effective amounts of one or more known lens care formulation components such as a detergent or surfactant component, a secondary comfort or wetting agent, a chelating or sequestering component, a buffer or a tonicity component.

Suitable surfactants can be either amphoteric or nonionic, and are typically present (individually or in combination) in amounts up to about 2% (w/v). The surfactant should be soluble in the lens care solution and non-irritating to ocular tissues. The presence of nonionic surfactants comprising one or more chains or polymeric components having oxyalkylene (-O--R--) repeats units wherein R has 2 to 6 carbon atoms are common to lens care solutions. Satisfactory non-ionic surfactants include polyethylene glycol esters of fatty acids, e.g. coconut, polysorbate, polyoxyethylene or polyoxypropylene ethers of higher alkanes (C₁₂-C₁₈). Examples of this class include polysorbate 20 (available under the trademark Tween® 20), polyoxyethylene (23) lauryl ether (Brij® 35), polyoxyethyene (40) stearate (Myrj®52), polyoxyethylene (25) propylene glycol stearate (Atlas® G 2612). Still other preferred surfactants include tyloxapol, polysulfates, polyethylene glycol, alkyl esters and any mixture thereof. The foregoing surfactants will generally be present in a total amount from 0. 1% to 2% (w/v), or from 0.1 % to 1.0% (w/v). Often the amount of surfactant is from 0.005% or 0.01%, to 0.1% or 0.5% or 0.8% (w/v).

A particular non-ionic surfactant consisting of a poly(oxypropylene)-poly(oxyethylene) adduct of ethylene diamine having a molecular weight from about 7,500 to about 27,000 wherein at least 40 weight percent of said adduct is poly(oxyethylene) has been found to be particularly advantageous for use in cleaning and conditioning both soft and hard contact lenses when used in amounts from about 0.05 to about 2.0 wt.%. The CTFA Cosmetic Ingredient Dictionary's adopted name for this group of surfactants is poloxamine. Such surfactants are available from BASF Wyandotte Corp., Wyandotte, Mich., under Tetronic®. Particularly good results are obtained with Tetronic®1107, Tetronic®1304 and Tetronic®904.

An analogous of series of surfactants, for use in the lens care compositions, is the poloxamer series which is a poly(oxyethylene) poly(oxypropylene) block polymers available under Pluronic® (commercially available form BASF). In accordance with one embodiment of a lens care composition the poly(oxyethylene)-poly(oxypropylene) block copolymers will have molecular weights from 2500 to 13,000 daltons or from 6000 to about 12,000 daltons. Specific examples of surfactants which are satisfactory include: poloxamer 108, poloxamer 188, poloxamer 237, poloxamer 238, poloxamer 288 and poloxamer 407. Particularly good results are obtained with poloxamer 237.

The amphoteric surfactants of general formula I are surface-active compounds with both acidic and alkaline properties. The amphoteric surfactants of general formula I include a class of compounds known as betaines. The betaines are characterized by a fully quaternized nitrogen atom and do not exhibit anionic properties in alkaline solutions, which means that betaines are present only as zwitterions at near neutral pH. An amphoteric surfactant of general formula I 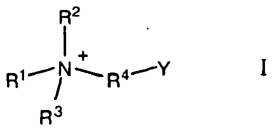 wherein R¹ is R or -(CH₂)ₙ-NHC(O)R, wherein R is a C₈-C₃₀alkyl optionally substituted with hydroxyl and n is 2, 3 or 4; R² and R³ are each independently selected from the group consisting of hydrogen and C₁-C₄alkyl; R⁴ is a C₂-C₈alkylene optionally substituted with hydroxyl; and Y is CO₂⁻ or SO₃⁻, can be present in the ophthalmic compositions, typically from 0.01 wt.% to 2 wt.%. Often the amount of amphoteric surfactant is from 0.005% or 0.01 %, to 0.1% or 0.5% or 0.8% (w/v).

All betaines are characterized by a fully quaternized nitrogen. In alkyl betaines, one of the alkyl groups of the quaternized nitrogen is an alkyl chain with eight to thirty carbon atoms. One class of betaines is the sulfobetaines or hydroxysulfobetaines in which the carboxylic group of alkyl betaine is replaced by sulfonate. In hydroxysulfobetaines a hydroxy-group is positioned on one of the alkylene carbons that extend from the quaternized nitrogen to the sulfonate. In alkylamido betaines, an amide group is inserted as a link between the hydrophobic C₈-C₃₀alkyl chain and the quaternized nitrogen.

In many embodiments, the amphoteric surfactant of general formula I is a sulfobetaine of general formula II 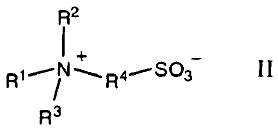 wherein R¹ is a C₈-C₃₀alkyl; R² and R³ are each independently selected from a C₁-C₄alkyl; and R⁴ is a C₂-C₈alkylene.

Certain sulfobetaines of general formula II are more preferred than others. For example, Zwitergent®3-10 available from Calbiochem Company, is a sulfobetaine of general formula I wherein R¹ is a straight, saturated alkyl with ten (10) carbons, R² and R³ are each methyl and R⁴ is -CH₂CH₂CH₂- (three carbons, (3)). Other sulfobetaines that can be used in the ophthalmic compositions include the corresponding Zwitergent®3-08 (R¹ is a is a straight, saturated alkyl with eight carbons), Zwitergent®3-12 (R¹ is a is a straight, saturated alkyl with twelve carbons), Zwitergent®3-14 (R¹ is a is a straight, saturated alkyl with fourteen carbons) and Zwitergent®3-16 (R¹ is a is a straight, saturated alkyl with sixteen carbons). Accordingly, some of the more preferred the ophthalmic composition will include a sulfobetaine of general formula II wherein R¹ is a C₈-C₁₆alkyl and R² and R³ is methyl.

In another embodiment, the amphoteric surfactant of general formula I is a hydroxysulfobetaine of general formula III 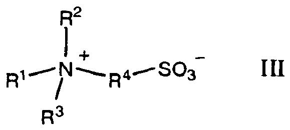 wherein R¹ is a C₈-C₃₀alkyl substituted with at least one hydroxyl; R² and R³ are each independently selected from a C₁-C₄alkyl; and R⁴ is a C₂-C₈alkylene substituted with at least one hydroxyl.

In another embodiment, the amphoteric surfactant is an alkylamido betaine of general formula IV 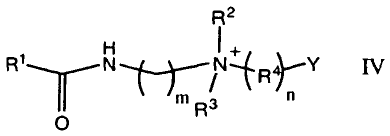 wherein R¹ is a C₈-C₃₀alkyl, and m and n are independently selected from 2, 3, 4 or 5; R² and R³ are each independently selected from a C₁-C₄alkyl optionally substituted with hydroxyl; R⁴ is a C₂-C₈alkylene optionally substituted with hydroxyl; and Y is CO₂⁻ or SO₃⁻. The most common alkylamido betaines are alkylamidopropyl betaines, e.g., cocoamidopropyl dimethyl betaine and lauroyl amidopropyl dimethyl betaine.

In addition to removing contaminants from the lens, the presence of an amphoteric surfactant of general formula I appears to counter the interaction between the hyaluronic acid and both PHMB and polyquaternium-1. The result is a lens care solution that exhibits exceptional biocidal activity and biocidal stability over time with minimal or little impact on the observed patient comfort profile that the hyaluronic acid provides. Accordingly, an amphoteric surfactant of general formula I is the surfactant of choice for the ophthalmic compositions.

The lens care solutions can also include a phosphonic acid, or its physiologically compatible salt, that is represented by the following formula: 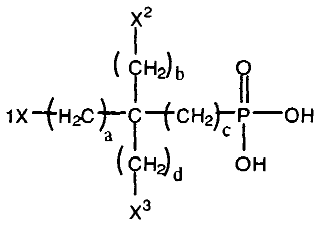 wherein each of a, b, c, and d are independently selected from integers from 0 to 4, preferably 0 or 1; X¹ is a phosphonic acid group (i.e., P(OH)₂O), hydroxy, amine or hydrogen; and X² and X³ are independently selected from the group consisting of halogen, hydroxy, amine, carboxy, alkylcarbonyl, alkoxycarbonyl, or substituted or unsubstituted phenyl, and methyl. Exemplary substituents on the phenyl are halogen, hydroxy, amine, carboxy and/or alkyl groups. A particularly preferred species is that wherein a, b, c, and d in are zero, specifically the tetrasodium salt of 1-hydroxyethylidene-1,1-diphosphonic acid, also referred to as tetrasodium etidronate, commercially available from Monsanto Company as DeQuest® 2016 diphosphonic acid sodium salt or phosphonate.

The lens care solutions can also include dexpanthenol, which is an alcohol of pantothenic acid, also called Provitamin B5, D-pantothenyl alcohol or D-panthenol. In some formulations of the lens care compositions, dexpanthenol can exhibit good cleansing action and can stabilize the lachrymal film at the eye surface when placing a contact lens on the eye. Dexpanthenol is preferably present in the contact lens care compositions in an amount from 0.2% to 10% (w/v), from 0.5% to 5% (w/v), or from 1% to 3% (w/v).

The lens care solutions can also include sorbitol, which is a hexavalent sugar alcohol. Typically, dexpanthenol is used in combination with sorbitol. In specific formulations the combination dexpanthenol and sorbitol can provide enhanced cleansing action and can also stabilize the lachrymal film following placement of the contact lens on the eye. These formulations can substantially improve patient comfort when wearing contact lenses. Sorbitol is present in the lens care compositions in an amount from 0.4% to 10% (w/v), from 0.8% to 6% (w/v) or from 1% to 3% (w/v).

The lens care solutions can also include one or more neutral or basic amino acids. The neutral amino acids include: the alkyl-group-containing amino acids such as alanine, glycine, isoleucine, valine, leucine and proline; hydroxyl-group-containing amino acids such as serine, threonine and 4-hydroxyproline; thio-group-containing amino acids such as cysteine, methionine and asparagine. Examples of the basic amino acid include lysine, histidine and arginine. The one or more neutral or basic amino acids are present in the compositions at a total concentration of from 0.1% to 5% (w/v).

The lens care solutions can also include glycolic acid, asparatic acid or any mixture of the two at a total concentration of from 0.001% to 4% (w/v) or from 0.01% to 2.0% (w/v). In addition, the combined use of one or more amino acids and glycolic acid and/or asparatic acid can lead to a reduction in the change of the size of the contact lens due to swelling and shrinkage following placement of the lens on the eye. The stated combination provides a higher degree of compatibility with the contact lens compared to the absence of one of the two components in the composition.

The lens care solutions can also include glycolic acid, asparatic acid or any mixture of the two, in combination with 2-amino-2-methyl-1,3-propanediol or a salt thereof. In some cases, solutions that contain a mixture of two of the three, or all three, compounds minimize the change of the lens size following placement of the contact lens in the eye. The 2-amino-2-methyl-1,3-propanediol (AMPD) or the salt thereof is added to the solutions in an amount to satisfy a predetermined molar ratio of glycolic acid, asparatic acid or any mixture of the two and AMPD. The molar ratio of the two components glycolic acid and/or asparatic acid to AMPD is 1:20 to 1.3:1. The glycolic acid, asparatic acid or any mixture of the two is present in the compositions at a concentration of 0.01% to 5% (w/v) or at a concentration of 0.05% to 1% (w/v).

The contact lens care solutions will very likely include a buffer system. By the terms "buffer" or "buffer system" is meant a compound that, usually in combination with at least one other compound, provides a buffering system in solution that exhibits buffering capacity, that is, the capacity to neutralize, within limits, either acids or bases (alkali) with relatively little or no change in the original pH. Generally, the buffering components are present from 0.05% to 2.5% (w/v) or from 0.1% to 1.5% (w/v).

The term "buffering capacity" is defined to mean the millimoles (mM) of strong acid or base (or respectively, hydrogen or hydroxide ions) required to change the pH by one unit when added to one liter (a standard unit) of the buffer solution. The buffer capacity will depend on the type and concentration of the buffer components. The buffer capacity is measured from a starting pH of 6 to 8, preferably from 7.4 to 8.4.

Borate buffers include, for example, boric acid and its salts, for example, sodium borate or potassium borate. Borate buffers also include compounds such as potassium tetraborate or potassium metaborate that produce borate acid or its salt in solutions. Borate buffers are known for enhancing the efficacy of certain polymeric biguanides. For example, U.S. Pat. No. 4,758,595 to Ogunbiyi et al. describes that a contact-lens solution containing a polyaminopropyl biguanide (PAPB), also known as PHMB, can exhibit enhanced efficacy if combined with a borate buffer.

A phosphate buffer system preferably includes one or more monobasic phosphates, dibasic phosphates and the like. Particularly useful phosphate buffers are those selected from phosphate salts of alkali and/or alkaline earth metals. Examples of suitable phosphate buffers include one or more of sodium dibasic phosphate (Na₂HPO₄), sodium monobasic phosphate (NaH₂PO₄) and potassium monobasic phosphate (KH₂PO₄). The phosphate buffer components frequently are used in amounts from 0.01% or to 0.5% (w/v), calculated as phosphate ion.

Other known buffer compounds can optionally be added to the lens care compositions, for example, citrates, citric acid, sodium bicarbonate, TRIS, and the like. Other ingredients in the solution, while having other functions, may also affect the buffer capacity. For example, EDTA, often used as a complexing agent, can have a noticeable effect on the buffer capacity of a solution.

A preferred buffer system is based upon boric acid/borate or a combined boric/phosphate buffer system. For example a combined boric/phosphate buffer system can be formulated from a mixture of sodium borate and phosphoric acid, or the combination of sodium borate and the monobasic phosphate.

In a combined boric/phosphate buffer system, the solution comprises about 0.05 to 2.5% (w/v)of a phosphoric acid or its salt and 0.1 to 5.0% (w/v)of boric acid or its salt. The phosphate buffer is used (in total) at a concentration of 0.004 to 0.2 M (Molar), preferably 0.04 to 0.1 M. The borate buffer (in total) is used at a concentration of 0.02 to 0.8 M, preferably 0.07 to 0.2 M.

Another particular buffer system is based on diglycine. Diglycine can be used in the composition as the sole buffer system or in combination with another buffer system. The amount of diglycine or salts thereof in the composition is from 0.01 wt.% to 2 wt.%, 0.05 wt.% to 2 wt.%, 0.1 wt.% to 2 wt.% or from 0.1 wt.% to 0.5 wt.%.

The lens care solutions can also include one or more secondary comfort or wetting agents in addition to the hyaluronic acid. The secondary comfort agent can enhance and/or prolong the cleaning and wetting activity of the surfactant component and/or condition the lens surface rendering it more hydrophilic (less lipophilic) and/or to act as a demulcent on the eye.

Suitable secondary comfort or wetting agents include, but are not limited to, water soluble natural gums, cellulose-derived polymers and the like. Useful natural gums include guar gum, gum tragacanth and the like. Useful cellulose-derived comfort components include cellulose-derived polymers, such as hydroxypropyl cellulose, hydroxypropylmethyl cellulose, carboxymethyl cellulose, methyl cellulose, hydroxyethyl cellulose and the like. A very useful comfort component is hydroxypropylmethyl cellulose (HPMC). Some non-cellulose comfort components include propylene glycol or glycerin. The comfort components are typically present in the solution from 0.01% to 1% (w/v).

One preferred secondary comfort agent that is polyvinylpyrrolidone (PVP). PVP is a linear homopolymer or essentially a linear homopolymer comprising at least 90% repeat units derived from 1-vinyl-2-pyrrolidone monomer, the remainder of the monomer composition can include neutral monomer, e.g., vinyl or acrylates. Other synonyms for PVP include povidone, polyvidone, 1-vinyl-2-pyrolidinone, and 1-ethenyl-2-pyrolionone (CAS registry number 9003-39-8). The PVP will preferably have a weight average molecular weight from 10,000 to 250,000 or from 30,000 to 100,000. Such materials are sold by various companies, including ISP Technologies, Inc. under the trademark PLASDONE®K-29/32, from BASF under the trademark KOLLIDON®, for example, KOLLIDON® K-30 or K-90. It is also preferred that one use pharmaceutical) grade PVP.

The lens care solutions can also include one or more chelating components to assist in the removal of lipid and protein deposits from the lens surface following daily use. Typically, the ophthalmic compositions will include relatively low amounts, e.g., from 0.005% to 0.05 % (w/v) of ethylenediaminetetraacetic acid (EDTA) or the corresponding metal salts thereof such as the disodium salt, Na₂EDTA.

One possible alternative to the chelator Na₂EDTA or a possible combination with Na₂EDTA, is a disuccinate of formula IV below or a corresponding salt thereof; 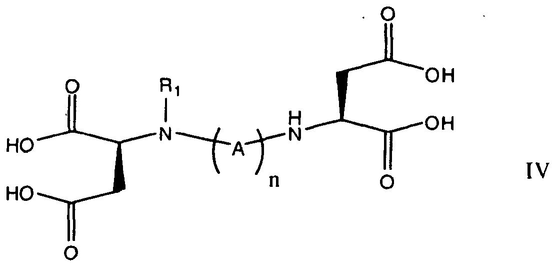 wherein R₁ is selected from hydrogen, alkyl or -C(O)alkyl, the alkyl having one to twelve carbons and optionally one or more oxygen atoms, A is a methylene group or an oxyalkylene group, and n is from 2 to 8. In one embodiment, the disuccinate is S,S-ethylenediamine disuccinate (S,S-EDDS) or a corresponding salt thereof. One commercial source of S,S-EDDS is represented by Octaquest® E30, which is commercially available from Octel. The chemical structure of the trisodium salt of S,S-EDDS is shown below. The salts can also include the alkaline earth metals such as calcium or magnesium. The zinc or silver salt of the disuccinate can also be used in the ophthalmic compositions.

Still another class of chelators include alkyl ethylenediaminetriacetates such as nonayl ethylenediaminetriacetate. See, U.S. Patent No. 6,995,123 for a more complete description of such agents.

The lens care solutions will typically have an osmolality in the range of at least about 200 mOsmol/kg for example, about 300 or about 350 to about 400 mOsmol/kg. The lens care solutions are substantially isotonic or hypertonic (for example, slightly hypertonic) and are ophthalmically acceptable. Accordingly, the lens care solutions will typically include an effective amount of a tonicity adjusting component. Among the suitable tonicity adjusting components that can be used are those conventionally used in contact lens care products such as various inorganic salts. Sodium chloride and/or potassium chloride and the like are very useful tonicity components. The amount of tonicity adjusting component is effective to provide the desired degree of tonicity to the solution.

One exemplary ophthalmic composition is formulated as a contact lens disinfecting solution prepared with the components and amounts of each listed in Table 1.

**Table 1.**

| **Component** | **Minimum Amount (wt.%)** | **Maximum Amount (wt.%)** | **Preferred Amount (wt.%)** |
|---|---|---|---|
| boric acid | 0.10 | 1.0 | 0.64 |
| sodium borate | 0.01 | 0.20 | 0.1 |
| sodium chloride | 0.20 | 0.80 | 0.49 |
| Zwitergent®3-10 | 0.005 | 0.5 | 0.05 |
| hyaluronic acid | 0.005 | 0.015 | 0.01 |
| Tetronic® 1107 | 0.05 | 2.0 | 1.00 |
| Na₂EDTA | 0.005 | 0.15 | 0.03 |
| PHMB-CG | 0.2 ppm | 3 ppm | 1.3 ppm |
| polyquaternium-1 | 0.5 ppm | 5 ppm | 1 ppm |

Another contact lens solution according to the present invention includes the following ingredients listed in Table 2.

**Table 2.**

| **Component** | **Minimum Amount (wt.%)** | **Maximum Amount (wt.%)** | **Preferred Amount (wt.%)** |
|---|---|---|---|
| sorbitol or xylitol | 0.5 | 5 | 3 |
| poloxamer 407 | 0.05 | 1.0 | 0.10 |
| phosphate monobasic | 0.10 | 0.8 | 0.46 |
| dexpanthenol | 0.01 | 1.0 | 0.03 |
| Zwitergent®3-10 | 0.01 | 0.2 | 0.05 |
| hyaluronic acid | 0.005 | 0.015 | 0.01 |
| Na₂EDTA | 0.005 | 0.3 | 0.1 |
| PHMB-CG | 0.2 ppm | 2 ppm | 1 ppm |

Another contact lens solution according to the present invention includes the following ingredients listed in Table 3.

**Table 3.**

| **Component** | **Minimum Amount (wt. %)** | **Maximum Amount (wt.%)** | **Preferred Amount (wt.%)** |
|---|---|---|---|
| NaCl/KCl | 0.2 | 1.0 | 0.50 |
| propylene glycol | 0.1 | 1.0 | 0.50 |
| poloxamer 237 | 0.01 | 0.20 | 0.05 |
| phosphate monobasic | 0.05 | 0.40 | 0.10 |
| phosphate dibasic | 0.05 | 0.4 | 0.12 |
| Hyaluronic acid | 0.005 | 0.015 | 0.008 |
| Na₂EDTA | 0.005 | 0.3 | 0.1 |
| PHMB-CG | 0.2 ppm | 2ppm. | 1.1 ppm |
| polyquaternium-1 | 0.5 ppm | 5 ppm | 1 ppm |

Another contact lens solution according to the present invention includes the following ingredients listed in Table 4.

**Table 4**

| **Component** | **Minimum Amount (wt.%)** | **Maximum Amount (wt.%)** | **Preferred Amount (wt.%)** |
|---|---|---|---|
| NaCl/KCl | 0.05 | 0.5 | 0.10 |
| phosphate monobasic | 0.05 | 0.40 | 0.12 |
| phosphate dibasic | 0.05 | 0.4 | 0.21 |
| sorbitol | 0.5 | 2.0 | 1.0 |
| Tetronic®904 | 0.02 | 0.5 | 0.10 |
| Povidone K90 | 0.05 | 0.5 | 0.10 |
| hyaluronic acid | 0.005 | 0.015 | 0.01 |
| Na₂EDTA | 0.005 | 0.3 | 0.1 |
| PHMB-CG | 0.2 ppm | 2 ppm | 1 ppm |

As described, the ophthalmic compositions can be used to clean and disinfect contact lenses. In general, the contact lens solutions can be used as a daily or every other day care regimen known in the art as a "no-rub" regimen. This procedure includes removing the contact lens from the eye, rinsing both sides of the lens with a few milliliters of solution and placing the lens in a lens storage case. The lens is then immersed in fresh solution for at least two hours. The lens is the removed from the case, optionally rinsed with more solution, and repositioned on the eye.

Alternatively, a rub protocol would include each of the above steps plus the step of adding a few drops of the solution to each side of the lens, followed by gently rubbing the surface between ones fingers for approximately 3 to 10 seconds. The lens can then be, optionally rinsed, and subsequently immersed in the solution for at least two hours. The lenses are removed from the lens storage case and repositioned on the eye.

The formulated contact lens solutions can be used with many different types of contact lenses including: (1) hard lenses formed from materials prepared by polymerization of acrylic esters, such as poly(methyl methacrylate) (PMMA), (2) rigid gas permeable (RGP) lenses formed from silicone acrylates and fluorosilicone methacrylates, (3) soft, hydrogel lenses, and (4) non-hydrogel elastomer lenses.

As an example, soft hydrogel contact lenses are made of a hydrogel polymeric material, a hydrogel being defined as a crosslinked polymeric system containing water in an equilibrium state. In general, hydrogels exhibit excellent biocompatibility properties, i.e., the property of being biologically or biochemically compatible by not producing a toxic, injurious or immunological response in a living tissue. Representative conventional hydrogel contact lens materials are made by polymerizing a monomer mixture comprising at least one hydrophilic monomer, such as (meth)acrylic acid, 2-hydroxyethyl methacrylate (HEMA), glyceryl methacrylate, N,N-dimethacrylamide, and N-vinylpyrrolidone (NVP). In the case of silicone hydrogels, the monomer mixture from which the copolymer is prepared further includes a silicone-containing monomer, in addition to the hydrophilic monomer. Generally, the monomer mixture will also include a crosslink monomer such as ethylene glycol dimethacrylate, tetraethylene glycol dimethacrylate, and methacryloxyethyl vinylcarbonate. Alternatively, either the silicone-containing monomer or the hydrophilic monomer may function as a crosslink agent.

The ophthalmic compositions can also be formulated for use as a preservative solution or packaging solution for contact lenses. One of ordinary skill in the art would know how to adjust the formulation for each of these respective applications. The term "preservative" or "to preserve" refers to the use of the compositions for the purpose of inhibiting the growth of microorganisms in a particular product, e.g., in an eye drop formulation.

The ophthalmic compositions can be used as a preservative in ophthalmic formulations for treating patients with dry eye. In such a method, the ophthalmic formulation is administered to the patient's eye, eye lid or to the skin surrounding the patient's eye. The formulation can be administered to the eyes irrespective of whether contact lenses are present in the eyes of the patient. For example, many people suffer from temporary or chronic eye conditions in which the eye's tear system fails to provide adequate tear volume or tear film stability necessary to remove irritating environmental contaminants such as dust, pollen, or the like.

Alternatively, the ophthalmic compositions can be used as a preservative in ophthalmic formulations for treating an ocular disease or ocular condition. In many instances, the ophthalmic compositions will include one or more active pharmaceutical agents. Generally, the active pharmaceutical agent is in one or more classes of ocular pharmaceuticals including, but not limited to anti-inflammatory agents, antibiotics, immunosuppressive agents, antiviral agents, antifungal agents, anesthetics and pain killers, anticancer agents, anti-glaucoma agents, peptide and proteins, anti-allergy agents.

### Examples

Hexamethylene bis(cyanoguanidine) (HMBDA) is prepared according to the method described in U.S. Patent No. 5,965,088 (Example 1).

### ¹³C NMR Pulse Sequence and Acquisition Parameters

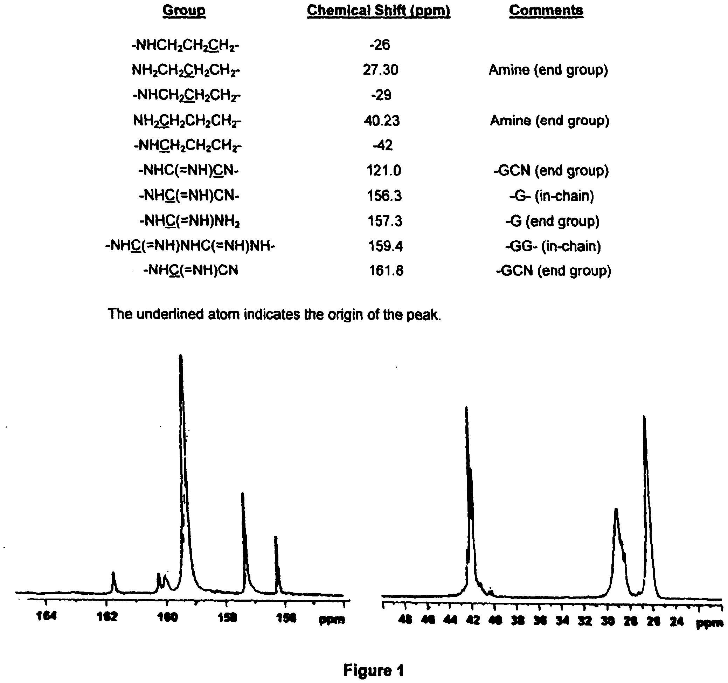

The resulting polymeric biguanide compositions provided by the Examples 1 to 5 above are analyzed by ¹³C NMR to determine the molar concentration of terminal end groups in each Example composition. The special pulse technique used to acquire the ¹³C spectra allows one to quantify the relative concentration of each terminal end group, that is, a guanidine, a cyanoguanidino or an amine. The ¹³C NMR data is also used to quantify the relative concentrations of in-chain biguanide groups and in-chain guanide. A representative ¹³C NMR spectrum of one of the polymeric biguanides of the invantioin is shown in Figure 1. As indicated, the alpha-methylene carbon associated with the terminal amine group is indicated by peak A, the guanidine carbon associated with the terminal guanidine group is indicated by peak B and the guanidine carbon associated with the terminal cyanoguanidino group is indicated by peak C. Also, the carbon associated with the in-chain biguanide is indicated by peak D, and the carbon associated with the in-chain guanide is indicated by peak E.

The samples for ¹³C NMR analysis are prepared using 2.2 ml of polymeric biguanide (20 wt%) in water and 0.3 ml D₂O is added. High-resolution ¹³C NMR is acquired using a Bruker AVANCE 300 MHz spectrometer operating at 75.5 MHz for ¹³C nuclei. For quantitative analysis, spectra are acquired using single-pulse excitation with inverse-gated decoupling for suppression of NOE effects, 1024 transients, and a relaxation delay that is five times longer than the longest ¹³C T₁ in the sample. At 300 MHz, the longest T₁ observed is 9.0 seconds for the terminal guanidine carbon at ∼ 157 ppm. A relaxation delay of 45 seconds is used to acquire quantitative spectra at 300 MHz. Since T₁'s are magnetic field dependent, it will be necessary to run a relaxation experiment if acquiring at a different field strength. All spectra are acquired at 300 K using a 10 mm BBO probe.

**Example 1.** PHMB (Cosmocil CG®, 6.0 g, 3.3 mmol), hexamethylene bis(cyanoguanido) (HMBDA) (0.9 g, 3.6 mmol) and concentrated hydrochloric acid (360 µL) are added to a reaction flask and heated to 100 °C until most of the liquid dissipates from the flask. The temperature of the reaction mixture is then heated to 155 °C for four hours. The reaction is allowed to cool overnight to room temperature over a flow of nitrogen. The resulting solids are dissolved in 50 mL of distilled water and solution purified by dialysis (100 MWCO tubing) overnight. The purified product is freeze dried, 4.81 g.

**Example 2.** PHMB (Cosmocil®CQ, 6.0 g, 3.3 mmol), hexamethylene bis(cyanoguanido) (HMBDA) (1.8 g, 7.2 mmol) and concentrated hydrochloric acid (720 µL) are added to a reaction flask and heated to 100 °C until most of the liquid dissipates from the flask. The temperature of the reaction mixture is then heated to 155 °C for four hours. The reaction is allowed to cool overnight to room temperature over a flow of nitrogen. The resulting solids are dissolved in 60 mL of distilled water and the solution purified by dialysis (100 MWCO tubing) overnight. The purified product is freeze dried, 5.3 g.

Examples I and 2 were analyzed by ¹³C NMR (see, below) to determine the molar concentration of terminal end groups. The ¹³C NMR data for Examples 1 and 2 along with commercial samples of PHMB are summarized in Table 5.

**Table 5.**

| **Example** | **Mₙ (GPC)** | **Mₙ (NMR)** | **terminal groups (mol%)** | | | **in-chain (mol%)** | |
|---|---|---|---|---|---|---|---|
| | | | **amine** | **CG** | **G** | **GG** | **G** |
| Cosmocil®CQ | 1568 | 1419 | 30.2 | 31.7 | 38.0 | 91.7 | 8.3 |
| Cosmocil®100 | 1695 | 1383 | 20.8 | 29.9 | 49.3 | 89.6 | 10.4 |
| 1 | 1392 | 1276 | 8.4 | 25.9 | 65.7 | 89.4 | 10.6 |
| 2 | 1089 | 829 | 0 | 11.7 | 88.3 | 84.3 | 15.7 |

### Examples 3A to 3C.

For each of the preparations, PHMB. (Cosmocil® 100, 6.0 g, 3.3 mmol) (Cosmocil®100 is a solid form of PHMB), hexamethylene bis(cyanoguanido) (HMBDA) (1.8 g, 7.2 mmol) and concentrated hydrochloric acid (720 µL) are added to a reaction flask and heated to 100 °C until most of the liquid dissipates from the flask. The temperature of the reaction mixture is then heated to 155 °C for four hours. The reaction is allowed to cool overnight to room temperature over a flow of nitrogen. The resulting solids are dissolved in 60 mL of distilled water and the olution purified by dialysis (100 MWCO tubing) overnight. The purified product is then freeze dried overnight.

The ¹³C NMR data for Examples 3A to 3C are summarized in Table 6.

**Table 6.**

| **Example** | **Mₙ (GPC)** | **Mₙ (NMR)** | **terminal groups (mol%)** | | | **in-chain (mol%)** | |
|---|---|---|---|---|---|---|---|
| | | | **amine** | **CG** | **G** | **GG** | **G** |
| 3A | 1808 | 1466 | 9.5 | 20.0 | 70.5 | 86.9 | 13.1 |
| 3B | 1758 | 1460 | 7.5 | 23.0 | 69.5 | 87.9 | 12.1 |
| 3C | 1751 | 1449 | 11.5 | 20.0 | 68.5 | 87.2 | 12.8 |

### Examples 4A to 4D.

For each of the preparations, PHMB (Cosmocil®100, 6.0 g, 3.3 mmol) (Cosmoci®100 is a solid form of PHMB), hexamethylene bis(cyanoguanido) (HMBDA) (1.8 g, 7.2 mmol) and concentrated hydrochloric acid (720 µL) are added to a reaction flask and heated to 100 °C until most of the liquid dissipates from the flask. The temperature of the reaction mixture is then heated to 155°C for one hour (Example 4A), two hours (Example 4B), three hours (Example 4C) and four hours (Example 4D). The reaction is allowed to cool overnight to room temperature over a flow of nitrogen. The resulting solids are dissolved in 60 mL of distilled water and the solution purified by dialysis (100 MWCO tubing) overnight. The purified product is then freeze dried overnight. The ¹³C NMR data for Examples 4A to 4D are summarized in Table 7.

**Table 7.**

| **Example** | **Mₙ (GPC)** | **Mₙ (NMR)** | **terminal groups (mol%)** | | | **in-chain (mol %)** | |
|---|---|---|---|---|---|---|---|
| | | | **amine** | **CG** | **G** | **GG** | **G** |
| 4A* | 1581 | 1593 | 15.4 | 33.8 | 50.7 | 89.6 | 10.4 |
| 4B | 1679 | 1444 | 13.9 | 27.9 | 58.2 | 89.0 | 11.0 |
| 4C | 2036 | 1515 | 10.5 | 32.5 | 57.0 | 88.9 | 11.1 |
| 4D | 2002 | 1437 | 9.5 | 28.1 | 62.3 | 88.5 | 11.5 |

| | | | | | | | |
|---|---|---|---|---|---|---|---|
| *Example 4A is not according to the invention. | | | | | | | |

### Example 5.

An aqueous solution containing sodium dicyanimide (8.9 g), hexamethylene (11.6 g), HMBDA (8.9 g), 36% hydrochloric acid (19 g) and water (7.3 g) is prepared with a pH from 6.5 to 7.5. The solution is heated to 120 °C to remove all of the water. The reaction vessel is then heated to 150 °C and this temperature is maintained for four hours. The reaction is cooled overnight under nitrogen. The resulting solids are dissolved in 60 mL of distilled water and the solution purified by dialysis (100 MWCO tubing) overnight. The purified product is then freeze dried overnight. A biguanide product comprising less than 18 mol% of terminal amine groups and 40 mol% and greater of terminal cyanoguanidino groups is obtained as measured by ¹³C NMR.

### Example 6A. Multipurpose Solution Formulation

A multipurpose solution were formulated with the components and amounts listed in Table 8A. A compounding vessel was charged with 85 to 90 percent of the batch weight in purified water. The following materials were then added in the order listed: sodium chloride, edentate disodium, boric acid, sodium borate, hydroxyalkyl phosphonate (Dequest® 30%), and Tetronic® 1107 and the solution stirred for not less than 10 mins. The sodium hyaluronate was added to the solution at a temperature not less than 70°C with stirring, and the solution was stirred for not less than 20 mins. The pH with was adjusted with 1N NaOH or 1N HCl if required. The solution was put through a sterilization cycle: Autoclave, 30-40 min at 121-124 °C; cool batch to less than40 °C.

An appropriate volume of purified water was charged to a second compounding vessel with a 20 %w/w PAPB-CG hydrochloride solution. The biguanide solution was mixed for not less than 10 mins, and transferred to the main compounding tank through a sterilizing filter (0.22µm). An additional amount of water is added to bring to batch weight, and the final solution stirred for not less than 15 mins.

**Table 8A.**

| **Component** | **w/w%** |
|---|---|
| boric acid | 0.64 |
| sodium borate | 0.11 |
| Dequest® 30% | 0.1 |
| Na₂EDTA | 0.11 |
| Tetronic®1107 | 1.0 |
| hyaluronic acid Na salt^{a} | 0.02 |
| PHMB-CG (Ex. 5) | 0.8 ppm |
| sodium chloride | 0.5 |
| purified water | Q.S. to 100% w/w |

| | |
|---|---|
| ^{a} Commercial product from Shandong Freda Biochem Co. Ltd. | |

**Example 6B.** A multipurpose solution is formulated with the components and amounts listed in Table 8B using the procedure described for Example 6A with the exception that cetylpyridinium chloride is added to the second compounding vessel instead of the PHMB.

**Table 8B.**

| **Component** | **w/w%** |
|---|---|
| Tris HCl | 0.055 |
| Tris base | 0.021 |
| Dequest® 30% | 0.1 |
| Na₂EDTA | 0.01 |
| Tetronic®1107 | 1.0 |
| hyaluronic acid Na salt^{a} | 0.02 |
| cetylpyridinium chloride | 2 ppm |
| propylene glycol | 0.3 |
| sodium chloride | 0.5 |
| purified water | Q.S.to 100% w/w |

| | |
|---|---|
| ^{a} Commercial product from Shandong Freda Biochem Co. Ltd. | |

**Example 6C.** A multipurpose solution is formulated with the components and amounts listed in Table 8C using the procedure described for Example 6A with the exception that the cetylpyridinium chloride is added to the second compounding vessel in combination with PHMB-CG.

**Table 8C.**

| **Component** | **w/w %** |
|---|---|
| boric acid | 0.64 |
| sodium borate | 0.11 |
| Dequest® 30% | 0.1 |
| Na₂EDTA | 0.05 |
| Tetronic® 1107 | 1.0 |
| hyaluronic acid Na salt^{a} | 0.02 |
| cetylpyridinium chloride | 0.6 ppm |
| PHMB-CG (Ex. 5) | 0.8 ppm |
| propylene glycol | 0.2 |
| sodium chloride | 0.5 |
| purified water | Q.S. to 100% w/w |

| | |
|---|---|
| ^{a} Commercial product from Shandong Freda Biochem Co. Ltd. | |

### Example7. Biocidal Efficacy without Organic Soil

The microbiocidal efficacy of the composition of Example 6A was evaluated after three (3) months at 25 °C and 40 °C based upon the performance requirement referred to as the "Stand-Alone Procedure for Disinfecting Products" Protocol GG 120706-3 as set the U.S. Food and Drug Administration, Division of Ophthalmic Devices. The microorganisms challenged in this procedure include: *Pseudomonas aeruginosa* (ATCC 9027), *Staphylococcus aureus* (ATCC 6538), *Serratia marcescens* (ATCC 13880), *Candida albicans* (ATCC 10231) and *Fusarium solani* (ATCC 36031). The log reduction of microorganisms determined from this testing for each formulation are shown in Table 9.

**Example 8.** The stand alone biocidal efficacy test described in Example 7 was also conducted in the presence of 10% organic soil and reported in Table 10.

**Table 9.**

| microorganism | time, hr | 25°C | 40 °C |
|---|---|---|---|
| S. Aureus | 1 | 3.0 | 3.0 |
| | 2 | >4.8 | 4.4 |
| | 3 | >4.8 | >4.8 |
| | 4 | >4.8 | >4.8 |
| | 24 | nd | nd |
| P. Aeruginosa | 1 | >4.8 | >4.8 |
| | 2 | >4.8 | >4.8 |
| | 3 | >4.8 | >4.8 |
| | 4 | >4.8 | >4.8 |
| | 24 | nd | nd |
| S. Marcescens | 1 | 3.9 | 4.8 |
| | 2 | >4.8 | >4.8 |
| | 3 | >4.8 | >4.8 |
| | 4 | >4.8 | >4.8 |
| | 24 | nd | nd |
| C. Albicans | 1 | 2.6 | 2.1 |
| | 2 | 3.2 | 2.5 |
| | 3 | 3.3 | 2.8 |
| | 4 | 3.1 | 2.8 |
| | 24 | 3.9 | 3.6 |
| F. Solani | 1 | 2.3 | 1.9 |
| | 2 | 2.3 | 2.1 |
| | 3 | 2.8 | 2.2 |
| | 4 | 2.8 | 2.3 |
| | 24 | 4.0 | 3.1 |

**Table 10.**

| microorganism | time,hr | 25 °C | 40 °C |
|---|---|---|---|
| S. Aureus | 1 | 3.5 | 2.8 |
| | 2 | 4.4 | >4.8 |
| | 3 | >4.8 | >4.8 |
| | 4 | >4.8 | >4.8 |
| | 24 | nd | nd |
| P. Aeruginosa | 1 | >4.6 | >4.6 |
| | 2 | >4.6 | >4.6 |
| | 3 | >4.6 | >4.6 |
| | 4 | >4.6 | >4.6 |
| | 24 | nd | nd |
| S. Marcescens | 1 | 2.2 | 2.2 |
| | 2 | 3.4 | 3.6 |
| | 3 | 4.4 | 4.5 |
| | 4 | >4.6 | >4.6 |
| | 24 | nd | nd |
| C. Albicans | 1 | 1.8 | 1.6 |
| | 2 | 2.5 | 1.8 |
| | 3 | 2.7 | 2.0 |
| | 4 | 2.7 | 2.1 |
| | 24 | 3.5 | 2.5 |
| F. Solani | 1 | 2.1 | 1.8 |
| | 2 | 2.6 | 2.2 |
| | 3 | 2.9 | 2.9 |
| | 4 | 3.2 | 3.2 |
| | 24 | >4.2 | >4.2 |

### Example 8.

A three-day, daily wear, dispensing study with twenty-three (23) patients was conducted to compare the clinical performance of the ophthalmic composition of Example 6A to Alcon's Opti-Free Replenish Multi-Purpose Solution, hereafter "Replenish", with PureVision lenses in contact lens wearers with a history of dry eye symptomology. The Example 6A solution soaked lenses exhibited statistically significant better comfort and less dryness at the in-office visits, and better morning visual quality scores compared to the Replenish soaked lenses. While the test solution soaked lenses demonstrated statistically significant less dryness compared to the control solution soaked lenses, the differences in dryness scores are not considered to be clinically significant.

There was also a trend for the Example 6A soaked PureVision lenses to exhibit better comfort (with the exception of mean morning comfort on Day 2) compared to Replenish soaked PureVision lenses. This is particularly seen at the end of Day 1 where the mean end-of-day comfort scores were 92.9 and 84.6 for the test and control solution soaked lenses, respectively; this difference is considered to be clinically significant.

There were no statistically significant differences noted between Example 6A and Replenish soaked lenses with respect to movement, inferior overlap, horizontal decentration, sting/burn , normalized visual acuity, deposition and wettability, normalized corneal and conjunctival staining, normalized bulbar and limbal injection, end-of day visual quality, morning and end-of-day comfort and lens cleanness, and forced choice preference for comfort.

### Clinical Procedure

A randomized, double-masked, repeated measures, contralateral eye study evaluation was conducted. Each well of the lens cases was pre-treated (a single, 4-hour minimum soak) with either Example 6A (test solution) or Replenish (control solution). For each case, the well treated with Example 6 solution was randomly determined and the opposite well received the Replenish. All Bausch & Lomb PureVision lenses were pre-treated (4-hour minimum soak), with either the Example 6A solution or Replenish in the pre-treated lens cases, following the same randomization used for the lens case wells.

Prior to lens insertion, bulbar and limbal injection, and corneal and conjunctival staining was assessed with the slit lamp. A spherical refraction was performed, through which high contrast/high illumination (HCHI), low contrast/high illumination (LCHI) and high contrast/low illumination (HCLI) visual acuity was measured. Each patient inserted a pre-treated test/control lens pair. Sting/bum and dryness were immediately rated. After the lenses settled, each lens was evaluated for movement, centration, comfort, wettability and deposition according to methods well recognized in the art of evaluating contact lens solutioins.

A forced-choice preference for comfort was made. A spherical over-refraction was performed, through which LogMAR visual acuity under all three testing conditions (HCHI, LCHI and HCLI) was measured. After approximately 2 hours of lens wear, each patient returned and the above tests were repeated, with the exception that the refraction and forced-preference for comfort was not repeated. Lenses were removed, corneal and conjunctival staining, and bulbar and limbal injection were reassessed, followed by reinsertion of the lenses.

Lenses were worn between eight and sixteen hours per day, for three days. Every evening, just prior to lens removal, patients recorded their daily wear time; and evaluated comfort, lens cleanness and visual quality. Patients were instructed to remove their right lens and thoroughly rinse each side of the lens for 5 seconds with the solution that was assigned for the right eye, place the contact lens in the lens case and fill with the same solution. This was repeated for the left lens with the solution assigned for the left eye. Upon daily lens insertion, patients evaluated comfort, lens cleanness and visual quality on the recording forms.

The patients returned on the fourth day. At the Day 4 (am) visit, the aforementioned tests were repeated. A forced-choice preference for comfort was completed. The lenses were stored in sensitive eyes saline. The 2-way repeated measures ANOVA was used to test for differences in each of the parametric dependent variables. Non-parametric data were analyzed using the Wilcoxon Matched Pairs test. Forced-choice data was assessed using the chi-square test. Differences at the alpha=0.05 level were considered statistically significant.

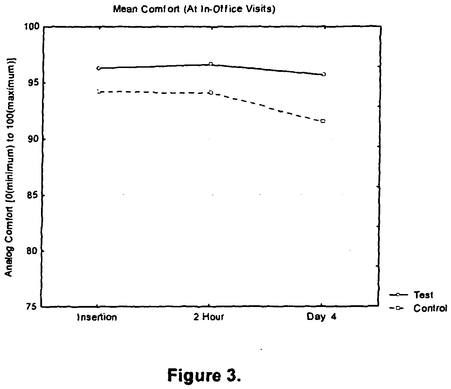

There was a statistically significant difference for mean comfort (measured at the in-office visits, ANOVA, p<0.04). The test solution soaked lenses exhibited better comfort compared to the control solution soaked lenses with means of 96.2 and 93.26, respectively (test = 96.3 at Insertion. 96.6 at 2 Hours and 95.7 at Day 4; control = 94.2 at Insertion, 94.1 at 2 Hours and 91.5 at Day 4), see FIG.3.

### Example 9. Lens Compatability Testing

The ophthalmic compositions also satisfied ISO Specifications for lens compatibility for several commercial contact lenses including PureVision®, Soflens 38®, Acuvue®, 02Optix® and Acuvue Advance®.

## Claims

1. An ophthalmic composition comprising:
a polymeric biguanide composition comprising less than 18 mol% of terminal amine groups, and 55 mol% or greater of terminal guanidine groups as measured by ¹³C-NMR; and 0.005 w/v% to 0.04 w/v% hyaluronic acid, or the metal salt thereof,
wherein the weight ratio of hyaluronic acid, or the metal salt thereof, to the polymeric biguanide composition is between 45:1 and 120:1.

2. The composition of claim 1 wherein the polymeric biguanide composition comprises less than 15 mol% of terminal amine groups, and 60 mol% or greater of terminal guanidine groups.

3. The composition of claim 2 wherein the polymeric biguanide composition comprises less than 10 mol% of terminal amine groups and 65 mol % or greater of terminal guanidine groups.

4. The composition of any one of claims 1 to 3 wherein the weight ratio of hyaluronic acid to the polymeric biguanide composition in the composition is from 55:1 to 90:1.

5. The composition of any one of claims 1 to 4 further comprising 0.01 wt.% to 0.8 wt.% of an amphoteric surfactant of general formula I 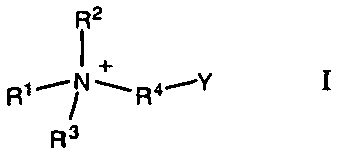 wherein R¹ is R or -(CH₂)ₙ-NHC(O)R, wherein R is a C₈-C₃₀-alkyl optionally substituted with hydroxyl and n is 2, 3 or 4; R² and R³ are each independently selected from the group consisting of hydrogen and C₁-C₄-alkyl; R⁴ is a C₂-C₈-alkylene optionally substituted with hydroxyl; and Y is CO₂⁻ or SO₃⁻

6. The composition of any one of claims 1 to 5 wherein the hyaluronic acid is present from 0.01 wt.% to 0.025 wt.%.

7. An ophthalmic composition comprising:
a polymeric biguanide composition comprising less than 18 mol% of terminal amine groups and 40 mol% or greater of terminal cyanoguanidino groups as measured by ¹³C-NMR; and
0.005 w/v% to 0.04 w/v% hyaluronic acid, or the metal salt thereof, wherein the weight ratio of hyaluronic acid, or the metal salt thereof,to the polymeric biguanide composition in the composition is from 55:1 to 90:1; and
an amphoteric surfactant of general formula I 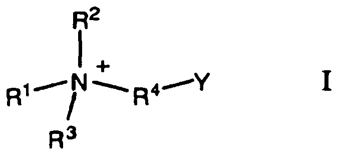 wherein R¹ is R or -(CH₂)ₙ-NHC(O)R, wherein R is a C₈-C₃₀-alkyl optionally substituted with hydroxyl and n is 2, 3 or 4; R² and R³ are each independently selected from the group consisting of hydrogen and C₁-C₄-alkyl; R⁴ is a C₂-C₈-alkylene optionally substituted with hydroxyl; and Y is CO₂⁻ or SO₃⁻.

8. The composition of claim 7 wherein the polymeric biguanide composition comprises less than 15 mol% of terminal amine groups and 50 mol% or greater of terminal cyanoguanidino groups.

9. The composition of claims 7 or 8 wherein the polymeric biguanide composition comprises from 10 mol% to 30 mol% of terminal guanidine groups.

10. The composition of any one of claims 1 to 9 wherein the hyaluronic acid is obtained via a fermentation mixture comprising Streptococcus equi.

11. The composition of any one of claims 1 to 10 wherein the hyaluronic acid is present from 0.01 wt.% to 0.025 wt.%.

12. The use of the composition of any one of claims 1 to 11 to clean, disinfect or package contact lenses, as a preservative in a pharmaceutical composition that includes a pharmaceutical agent, or as a preservative in a health care product.

13. A method of cleaning, disinfecting or packaging a contact lens comprising contacting said contact lens with the ophthalmic composition of any one of claims 1 to 11.

14. An ophthalmic composition comprising:
a polymeric biguanide composition comprising less than 18 mol% of terminal amine groups, and 40 mol% or greater of terminal cyanoguanidino groups as measured by ¹³C-NMR; and
0.005 w/v% to 0.04 w/v% hyaluronic acid, or the metal salt thereof,
wherein the weight ratio of hyaluronic acid, or the metal salt thereof, to the polymeric biguanide composition is between 45:1 and 120:1.

## Patentansprüche

1. Eine ophthalmische Zusammensetzung umfassend:
eine polymere Biguanid-Zusammensetzung, welche mittels ¹³C-NMR gemessen wurde, umfassend weniger als 18 Mol% einer terminalen Amin-Gruppe und 55 Mol% oder mehr einer terminalen Guanidin-Gruppe; und
0,005 w/v% bis 0,04 w/v% Hyaluronsäure oder des Metallsalzes davon,
wobei das Gewichtsverhältnis der Hyaluronsäure oder des Metallsalzes davon zur polymeren Biguanid-Zusammensetzung zwischen 45:1 und 120:1 liegt.

2. Die Zusammensetzung gemäß Anspruch 1, wobei die polymere Biguanid-Zusammensetzung weniger als 15 Mol% einer terminalen Amin-Gruppe und 60 Mol% oder mehr einer terminalen Guanidin-Gruppe umfasst.

3. Die Zusammensetzung gemäß Anspruch 2, wobei die polymere Biguanid-Zusammensetzung weniger als 10 Mol% einer terminalen Amin-Gruppe und 65 Mol% oder mehr einer terminalen Guanidin-Gruppe umfasst.

4. Die Zusammensetzung gemäß eines jeden der Ansprüche 1 bis 3, wobei das Gewichtsverhältnis der Hyaluronsäure oder des Metallsalzes davon zur polymeren Biguanid-Zusammensetzung in der Zusammensetzung zwischen 55:1 und 90:1 liegt.

5. Die Zusammensetzung gemäß eines jeden der Ansprüche 1 bis 4, weiterhin umfassend 0,01 Gew.% bis 0,8 Gew.% eines amphoteren Tensides der allgemeinen Formel I 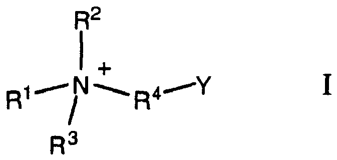 wobei R¹ R oder -(CH₂)ₙ-NHC(O)R ist, wobei R ein C₈-C₃₀-Alkyl ist, welches gegebenenfalls mit Hydroxyl substituiert ist und n 2, 3 oder 4 ist; R² und R³ jeweils unabhängig voneinander ausgewählt sind aus der Gruppe bestehend aus Wasserstoff und C₁-C₄-Alkyl; R⁴ ein C₂-C₈-Alkylen ist, welches gegebenenfalls mit Hydroxyl substituiert ist; und Y CO₂⁻ oder SO₃⁻ ist.

6. Die Zusammensetzung gemäß eines jeden der Ansprüche 1 bis 5, wobei die Hyaluronsäure von 0,01 Gew.% bis 0,025 Gew.% anwesend ist.

7. Eine ophthalmische Zusammensetzung umfassend:
eine polymere Biguanid-Zusammensetzung, welche weniger als 18 Mol% einer terminalen Amin-Gruppe und 40 Mol% oder mehr einer terminalen Cyanoguanidino-Gruppe, wie mit ¹³C-NMR gemessen, umfasst; und
0,005 w/v% bis 0,04 w/v% Hyaluronsäure oder des Metallsalzes davon, wobei das Gewichtsverhältnis der Hyaluronsäure oder des Metallsalzes davon zur polymeren Biguanid-Zusammensetzung zwischen 55:1 und 90:1 liegt; und
ein amphoteres Tensid der allgemeinen Formel I 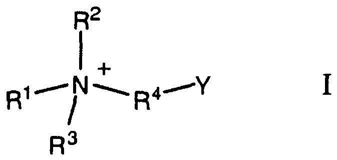 wobei R¹ R oder -(CH₂)ₙ-NHC(O)R ist, wobei R ein C₈-C₃₀-Alkyl ist, welches gegebenenfalls mit Hydroxyl substituiert ist und n 2, 3 oder 4 ist; R² und R³ jeweils unabhängig voneinander ausgewählt sind aus der Gruppe bestehend aus Wasserstoff und C₁-C₄-Alkyl; R⁴ ein C₂-C₈-Alkylen ist, welches gegebenenfalls mit Hydroxyl substituiert ist; und Y CO₂ oder SO₃⁻ ist.

8. Die Zusammensetzung gemäß Anspruch 7, wobei die polymere Biguanid-Zusammensetzung weniger als 15 Mol% einer terminalen Amin-Gruppe und 50 Mol% oder mehr einer terminalen Cyanoguanidino-Gruppe umfasst.

9. Die Zusammensetzung gemäß den Ansprüchen 7 oder 8, wobei die polymere Biguanid-Zusammensetzung 10 Mol% bis 30 Mol% einer terminalen Guanidin-Gruppe umfasst.

10. Die Zusammensetzung gemäß eines jeden der Ansprüche 1 bis 9, wobei die Hyaluronsäure über eine Fermentationsmischung, welche Streptococcus equi umfasst, erhalten wird.

11. Die Zusammensetzung gemäß eines jeden der Ansprüche 1 bis 10, wobei die Hyaluronsäure von 0,01 Gew.% bis 0,025 Gew.% anwesend ist.

12. Die Verwendung der Zusammensetzung gemäß eines jeden der Ansprüche 1 bis 11 zum Reinigen, Desinfizieren oder Verpacken von Kontaktlinsen, als ein Konservierungsmittel in einer pharmazeutischen Zusammensetzung, die einen pharmazeutischen Wirkstoff enthält oder als Konservierungsmittel in einem Medizinprodukt.

13. Ein Verfahren zum Reinigen, Desinfizieren oder Verpacken einer Kontaktlinse umfassend Kontaktieren besagter Kontaktlinse mit der ophthalmischen Zusammensetzung gemäß eines jeden der Ansprüche 1 bis 11.

14. Eine ophthalmische Zusammensetzung umfassend:
eine polymere Biguanid-Zusammensetzung, welche weniger als 18 Mol% einer terminalen Amin-Gruppe und 40 Mol% oder mehr einer terminalen Cyanoguanidino-Gruppe, wie mit ¹³C-NMR gemessen, umfasst; und
0,005 w/v% bis 0,04 w/v% Hyaluronsäure oder des Metallsalzes davon,
wobei das Gewichtsverhältnis der Hyaluronsäure oder des Metallsalzes davon zur polymeren Biguanid-Zusammensetzung zwischen 45:1 und 120:1 liegt.

## Revendications

1. Composition ophtalmique comprenant :
une composition de biguanide polymère comprenant moins de 18 % en moles de groupes amine terminaux, et 55 % en moles ou plus de groupes guanidine terminaux, tels que mesurés par RMN-¹³C ; et
de 0,005 % p/v à 0,04 % p/v d'acide hyaluronique, ou d'un sel métallique de celui-ci,
dans laquelle le rapport en poids de l'acide hyaluronique ou du sel métallique de celui-ci à la composition de biguanide polymère est compris entre 45/1 et 120/1.

2. Composition selon la revendication 1, dans laquelle la composition de biguanide polymère comprend moins de 15 % en moles de groupes amine terminaux et 60 % en moles ou plus de groupes guanidine terminaux.

3. Composition selon la revendication 2, dans laquelle la composition de biguanide polymère comprend moins de 10 % en moles de groupes amine terminaux et 65 % en moles ou plus de groupes guanidine terminaux.

4. Composition selon l'une quelconque des revendications 1 à 3, dans laquelle le rapport en poids de l'acide hyaluronique à la composition de biguanide polymère dans la composition est de 55/1 à 90/1.

5. Composition selon l'une quelconque des revendications 1 à 4, comprenant en outre de 0,01 % en poids à 0,8 % en poids d'un tensioactif amphotère de formule générale 1 : 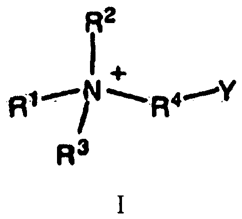 dans laquelle R¹ est R ou -(CH₂)ₙ-NHC(O)R où R est un alkyle en C₈ à C₃₀ éventuellement substitué par hydroxyle et n vaut 2, 3 ou 4 ; chacun de R² et R³ est indépendamment choisi dans le groupe constitué par l'hydrogène et alkyle en C₁ à C₄ ; R⁴ est un alkylène en C₂ à C₈ éventuellement substitué par hydroxyle ; et Y est CO₂⁻ ou SO₃⁻.

6. Composition selon l'une quelconque des revendications 1 à 5, dans laquelle l'acide hyaluronique est présent à raison de 0,01 % en poids à 0,025 % en poids.

7. Composition ophtalmique comprenant :
une composition de biguanide polymère comprenant moins de 18 % en moles de groupes amine terminaux, et 40 % en moles ou plus de groupes guanidine terminaux, tels que mesurés par RMN-¹³C ; et
de 0,005 % p/v à 0,04 % p/v d'acide hyaluronique, ou d'un sel métallique de celui-ci,
dans laquelle le rapport en poids de l'acide hyaluronique ou du sel métallique de celui-ci à la composition de biguanide polymère va de 55/1 à 90/1 ; et
un tensioactif amphotère de formule générale 1 : 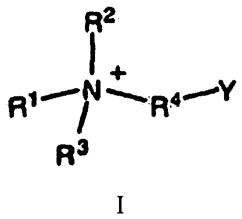 dans laquelle R¹ est R ou -(CH₂)ₙ-NHC(O)R où R est un alkyle en C₈ à C₃₀ éventuellement substitué par hydroxyle et n vaut 2, 3 ou 4 ; chacun de R² et R³ est indépendamment choisi dans le groupe constitué par l'hydrogène et alkyle en C₁ à C₄ ; R⁴ est un alkylène en C₂ à C₈ éventuellement substitué par hydroxyle ; et Y est CO₂⁻ ou SO₃⁻.

8. Composition selon la revendication 7, dans laquelle la composition de biguanide polymère comprend moins de 15 % en moles de groupes amine terminaux et 50 % en moles ou plus de groupes cyanoguanidine terminaux.

9. Composition selon la revendication 7 ou 8, dans laquelle la composition de biguanide polymère comprend de 10 % en moles à 30 % en moles de groupes guanidine terminaux.

10. Composition selon l'une quelconque des revendications 1 à 9, dans laquelle l'acide hyaluronique est obtenu via un mélange de fermentation comprenant Streptococcus equi.

11. Composition selon l'une quelconque des revendications 1 à 10, dans laquelle l'acide hyaluronique est présent à raison de 0,01 % en poids à 0,025 % en poids.

12. Utilisation de la composition de l'une quelconque des revendications 1 à 11 pour nettoyer, désinfecter ou conditionner des lentilles de contact, en tant que conservateur dans une composition pharmaceutique qui contient un agent pharmaceutique, ou en tant que conservateur dans un produit de soin de santé.

13. Procédé pour nettoyer, désinfecter ou conditionner une lentille de contact, comprenant la mise en contact de ladite lentille de contact avec la composition ophtalmique de l'une quelconque des revendications 1 à 11.

14. Composition ophtalmique comprenant :
une composition de biguanide polymère comprenant moins de 18 % en moles de groupes amine terminaux, et 40 % en moles ou plus de groupes guanidine terminaux, tels que mesurés par RMN- ¹³C ; et
de 0,005 % p/v à 0,04 % p/v d'acide hyaluronique, ou d'un sel métallique de celui-ci,
dans laquelle le rapport en poids de l'acide hyaluronique ou du sel métallique de celui-ci à la composition de biguanide polymère est compris entre 45/1 et 120/1.
